# EUROPEAN PATENT APPLICATION

(11) **EP 4 751 725 A1**
(43) Date of publication of application: **03.06.2026**
(21) Application number: 24845690.7
(22) Date of filing: 26.07.2024
(51) Int. Cl.: A61K 35/50, A61P 15/08, C12N 5/0735

(54) **AGENT FOR IMPROVING IMPLANTATION RATE AND METHOD FOR PRODUCING AGENT FOR IMPROVING IMPLANTATION RATE**

(30) Priority: 26.07.2023 JP 2023121523
(71) Applicant: Tohoku University, Sendai-shi, Miyagi 980-8577 (JP)
(72) Inventor: ARIMA, Takahiro, Sendai-shi, Miyagi 980-8577 (JP); SHIBATA, Shun, Sendai-shi, Miyagi 980-8577 (JP)
(74) Representative: Plasseraud IP
(86) International application number: PCT/JP2024/026757
(87) International publication number: WO 2025/023316

(57) **Abstract**

Provided is an agent for improving an implantation rate, the agent containing a culture supernatant of a trophoblast stem cell. Provided is a pharmaceutical composition for treating or preventing implantation failure, the pharmaceutical composition containing the agent for improving an implantation rate. Provided is a method for producing an agent for improving an implantation rate, the method including (i) a step of culturing a trophoblast stem cell, and (ii) a step of obtaining a culture supernatant from a culture obtained by the culturing.

## Description

### Technical Field

The present invention relates to an agent for improving an implantation rate and a method for producing an agent for improving an implantation rate.

The present application claims priority to Japanese Patent Application No. 2023-121523 filed in Japan on July 26, 2023, the contents of which are incorporated herein.

### Background Art

Assisted reproductive technologies are on the rise every year, but an average success rate for in vitro fertilization and intracytoplasmic sperm injection is approximately 20% to 25%. Among patients who do not achieve pregnancy, the number of patients suspected to be caused by implantation failure remains high. Therefore, there is a need to develop technologies for improving an implantation rate.

As a method for improving the implantation rate, a method called Stimulation of Endometrium-Embryo Transfer (SEET method) has been reported (for example, PTL 1). The SEET method is a method in which a supernatant of a culture solution of embryos cultured until a blastocyst state is injected into the uterus 2 days to 3 days before embryo transfer.

A main component of the placental tissue is the trophoblast tissue. Inventors of the present invention have developed a method of collecting CD49f antibody-positive cells from a cell suspension obtained from a mammalian placental tissue and establishing trophoblast stem cells (TS cells) (PTL 2). The inventors have further developed a method of inducing TS cells from pluripotent stem cells (PTL 3) and a method of inducing TS cells from placenta-derived trophoblast cells from mid-pregnancy onward (PTL 4).

### Citation List

### Patent Literature

PTL 1: JP5139294B
PTL 2: JP6400832B
PTL 3: WO2020/250438
PTL 4: WO2022/014028

### Summary of Invention

### Technical Problem

In order to increase the success rate of pregnancy in the assisted reproductive technologies, there is a need to develop a technology that improves the implantation rate. In the SEET method, an amount of the culture supernatant obtained by culturing blastocysts is limited, and an amount that can be injected into the uterus is extremely small. Therefore, there are technical and procedural problems, and currently this technology is only used in a few clinics.

Therefore, an object of the invention is to provide an agent for improving an implantation rate that can be easily produced, a pharmaceutical composition containing the agent for improving an implantation rate, and a method for producing the agent for improving an implantation rate.

### Solution to Problem

The invention includes the following aspects.
[1] An agent for improving an implantation rate, the agent containing a culture supernatant of a trophoblast stem cell.
[2] The agent for improving an implantation rate according to [1], which is for use in treating an endometrium before embryo transfer.
[3] The agent for improving an implantation rate according to [1] or [2], in which the culture supernatant is a culture supernatant obtained from a culture of the trophoblast stem cell cultured in a serum-free medium.
[4] The agent for improving an implantation rate according to any one of [1] to [3], in which the culture supernatant is a culture supernatant obtained from a culture of the trophoblast stem cell cultured for 1 day to 10 days.
[5] A pharmaceutical composition for treating or preventing implantation failure, the pharmaceutical composition containing the agent for improving an implantation rate according to any one of [1] to [4].
[6] A method for producing an agent for improving an implantation rate, the method including (i) a step of culturing a trophoblast stem cell, and (ii) a step of obtaining a culture supernatant from a culture obtained by the culturing.
[7] The method for producing an agent for improving an implantation rate according to [6], in which the culturing in the step (i) is performed in a serum-free medium.
[8] The method for producing an agent for improving an implantation rate according to [6] or [7], in which the culturing in the step (i) is performed for 1 day to 10 days.

### Advantageous Effects of Invention

According to the present invention, there are provided an agent for improving an implantation rate that can be easily produced, a pharmaceutical composition containing the agent for improving an implantation rate, and a method for producing the agent for improving an implantation rate.

### Brief Description of Drawings

[FIG. 1] FIG. 1 is an image of human TS cells (GFP-TS) cells that constitutively express EGFP and endometrial epithelial organoids (EMOs) three-dimensionally co-cultured in Matrigel, in which a left side of the image is a bright field image, a right side of the image is a fluorescence microscope image detecting EGFP fluorescence, an arrow points to a thickened epithelial cell layer, and the scale bar is 100 µm.
[FIG. 2] FIG. 2 is an immunostaining image of human TS cells (GFP-TS) cells that constitutively express EGFP and endometrial epithelial organoids (EMOs) three-dimensionally co-cultured in Matrigel, in which arrows point to human TS cell, and the scale bar is 100 µm.
[FIG. 3] FIG. 3 includes a lower diagram showing results of gene expression analysis of implantation marker genes (PAEP, SPP1) in endometrial models treated with a culture supernatant of human TS cells (TS-CM), a culture supernatant pf human ST cells (ST-CM), or hormone (EPCP), and an upper diagram showing an outline of a test method, in which Control represents an untreated group, and Hormone represents a group added with EPCP (E₂ (10 nM), MPA (1 µM), 8-Bromo-cAMP (50 µM) and prolactin (50 mg/mL)).
[FIG. 4A] FIG. 4A shows results of gene expression analysis of genes involved in endometrial receptivity analysis (ERA) in endometrial models treated with TS-CM, ST-CM, or Hormone.
[FIG. 4B] FIG. 4B shows results of GO term analysis of gene groups whose expression levels are elevated in the TS-CM-treated endometrial model.
[FIG. 5A] FIG. 5A is a phase contrast photograph showing adhesion of Blastoid to TS-CM-treated endometrial epithelial cells (Ishikawa cells), in which the scale bar is 200 µm.
[FIG. 5B] FIG. 5B shows results of quantifying adhesion rates of Blastoid after 4 hours of co-culturing of the TS-CM-treated endometrial epithelial cells (Ishikawa cells) and Blastoid, in which N = 12 Blastoids/group.

### Description of Embodiments

A numerical range expressed with "to" means a range that includes numerical values written before and after "to" as a lower limit value and an upper limit value.

A term "comprise" means that components other than the target component may be included. A term "consist of" means that components other than the target component are not included. A term "consist essentially of" means that components other than the target component in an aspect that exerts a special function (such as an aspect that completely loses an effect of the invention) are not included. In the present description, when "comprise" is described, aspects of "consist of" and "consist essentially of" are included.

The cells may be isolated. The term "isolated" means a natural state or a state of being separated from other components. An "isolated" one may substantially not contain other components. The expression of "substantially not contain other components" means that a content of other components contained in an isolated component is negligible. The content of other components contained in the isolated component may be, for example, 10 mass% or less, 5 mass% or less, 4 mass% or less, 3 mass% or less, 2 mass% or less, 1 mass% or less, 0.5 mass% or less, or 0.1 mass% or less. The cells described in the description can be isolated cells.

### [Agent for Improving Implantation Rate]

A first aspect of the present disclosure is an agent for improving an implantation rate. The agent for improving an implantation rate contains a culture supernatant of trophoblast stem cells.

### <Trophoblast Stem Cells (TS Cells)>

TS cells are cells that have an ability to differentiate into cells that make up the placenta. More specifically, TS cells have an ability to differentiate into extravillous cytotrophoblast cells (EVT) and syncytiotrophoblast cells (ST). A differentiation induction test into EVT and a differentiation induction test into ST can be performed by a known method (for example, JP6400832B, WO2020/250438, WO2022/014028 or the like).

Examples of a positive marker of TS cells include GATA2, GATA3, TFAP2, ELF5, and ZNF750. Examples of a negative marker of TS cells include CDX2. TS cells can be said to be cells that have the ability to differentiate into EVT and ST and are positive for at least one (preferably two or more, more preferably three or more, still more preferably four or more, and particularly preferably five) selected from the group consisting of GATA2, GATA3, TFAP2, ELF5, and ZNF750. Alternatively, TS cells can be said to be cells that are negative for CDX2 in addition to the above. TS cells can also be said to be cells induced from blastocysts, CT cells, or pluripotent stem cells, which have the ability to differentiate into EVT and ST, and which are positive for at least one selected from the group consisting of GATA2, GATA3, TFAP2, ELF5, and ZNF750, and negative for CDX2.

TS cells can be prepared by known methods. TS cells may be induced from blastocysts, cytotrophoblast cells (CT cells), or pluripotent stem cells.

Examples of a method for inducing TS cells from blastocysts include the following one. For example, a placental tissue is appropriately subjected to a mechanical and/or enzymatic treatment to dissociate cells. The cells are then cultured in a culture medium that induces TS cells (for example, Okae et al. Derivation of Human Trophoblast Stem Cells. Cell Stem Cell (2018), 22(1), 50-63.e6.), and then TS cells can be established using expression of TS cell markers (GATA2-positive, GATA3-positive, TFAP2-positive, ELF5-positive, ZNF750-positive, CDX2-negative, and the like) as an indicator.

Examples of a method for inducing TS cells from CT cells include a method described in JP6400832B.

Examples of a method for inducing TS cells from pluripotent stem cells include a method described in WO2020/250438.

The biological species from which the TS cells are derived is preferably the same as the biological species to which the agent for improving an implantation rate is applied. For example, when the agent for improving an implantation rate is applied to humans, the TS cells are preferably human TS cells.

### <Culture Medium>

The culture medium for culturing TS cells may be any culture medium that allows maintenance culture of TS cells. Examples of the culture medium include a basal medium commonly used for culturing animal cells. The basal medium may contain components necessary for cell survival, such as amino acids, vitamins, and inorganic salts.

Examples of amino acids include glycine, alanine, arginine, asparagine, aspartic acid, cysteine, glutamic acid, glutamine, histidine, isoleucine, leucine, lysine, methionine, phenylalanine, proline, serine, threonine, tryptophan, tyrosine, and valine. These amino acids may be in the form of a salt such as a hydrochloride, or may be in the form of a salt hydrate.

Examples of vitamins include biotin, choline chloride, calcium pantothenate, folic acid, nicotinamide, para-aminobenzoic acid, pyridoxine, riboflavin, thiamine, vitamin B12, and inositol. These vitamins may be in the form of a salt such as a hydrochloride, or may be in the form of a salt hydrate.

Examples of inorganic salts include salts of alkali metals such as potassium and sodium, salts of alkaline earth metals such as calcium and magnesium, and salts of transition metals such as iron, copper, and zinc. These salts include hydrochlorides, nitrates, sulfates, phosphates, hydrogen phosphates, dihydrogen phosphates, carbonates, and hydrogen carbonates. Specific examples of inorganic salts include, but are not limited to, calcium chloride, copper sulfate, iron nitrate, iron sulfate, magnesium chloride, magnesium sulfate, potassium chloride, sodium bicarbonate, sodium chloride, sodium hydrogen phosphate, sodium dihydrogen phosphate, and zinc sulfate. These inorganic salts may be in the form of hydrates.

Other components contained in the basal medium include, but are not limited to, sugars such as glucose, peptides such as glutathione, purine derivatives such as hypoxanthine, fatty acids such as linoleic acid, pH indicators such as phenol red, lipoic acid, putrescine, pyruvic acid, and thymidine.

Specific examples of the basal medium include Doulbecco's modified Eagle's Medium (DMEM) medium, DMEM/F12 medium, Advanced DMEM/F12 medium, IMDM medium, Medium 199 medium, Eagle's Minimum Essential Medium (EMEM) medium, αMEM medium, Ham's F12 medium, RPMI 1640 medium, Fischer's medium, and mixed media thereof. A preferred basal medium is, for example, DMEM/F12.

The culture medium may be a basal medium to which appropriate components are added. Components that can be added to the basal medium include, for example, serum and serum replacements. The culture medium is preferably a serum-free medium that does not contain serum. The culture medium is preferably a basal medium to which a serum replacement is added. The serum replacement is a substance or a composition that is added to a culture medium as a replacement for serum. Preferably, the serum replacement contains known components. Preferably, the serum replacement does not contain components derived from xenogeneic animals. The term "xenogeneic animal" refers to an animal of a different species from the animal species to which the agent for improving an implantation rate is applied.

Examples of the serum replacement include albumin, transferrin, sodium selenite, ITS-X (Invitrogen), Knockout Serum Replacement (KSR), N2 supplement (Invitrogen), B27 supplement (Invitrogen), fatty acids, insulin, collagen precursors, trace elements, 2-mercaptoethanol, 3'-thiolglycerol, L-ascorbic acid, and albumin. The serum replacement may be used alone or in combination of two or more kinds thereof. Preferred examples of the serum replacement include ITS-X supplement, KSR, L-ascorbic acid, and albumin (such as bovine serum albumin (BSA)).

The culture medium is preferably a culture medium obtained by adding a serum replacement to a basal medium, more preferably a culture medium obtained by adding at least one serum replacement selected from the group consisting of ITS-X supplement, KSR, L-ascorbic acid, and albumin (such as BSA) to a basal medium, and still more preferably a culture medium obtained by adding ITS-X supplement, KSR, L-ascorbic acid, and albumin (such as BSA) to a basal medium. As the basal medium, it is preferable to use the DMEM/F12 medium.

In addition to the components described above, the culture medium may contain components such as lipids, Glutamax, growth factors (such as EGF, FGF, and BMP), kinase inhibitors, signal transduction inhibitors, signal transduction activators, antibiotics, antioxidants, pyruvic acid, and buffers. The culture medium may be one that can maintain TS cells as they are without differentiating the TS cells (TS cell maintenance medium). In this case, it is preferable that the culture medium does not contain components that induce differentiation of TS cells (for example, growth factors, kinase inhibitors, signal transduction inhibitors, signal transduction activators, and differentiation inducers). The culture medium may be one that induces differentiation of TS cells (TS cell differentiation-inducing medium). In this case, the culture medium may contain components that induce differentiation of TS cells into EVT or ST (for example, growth factors, kinase inhibitors, signal transduction inhibitors, signal transduction activators, and differentiation inducers). The culture medium for inducing differentiation of TS cells into EVT may contain a growth factor such as neuregulin (NRG) 1, a TGFβ inhibitor such as A83-01, and a ROCK inhibitor such as Y27632. The culture medium for inducing differentiation of TS cells into ST cells may contain 2-mercaptoethanol, a ROCK inhibitor such as Y27632, and forskolin. The culture medium preferably does not contain components derived from xenogeneic animals, and is preferably xeno-free.

Specific examples of a preferred culture medium include the DMEM/F12 medium and the "basal medium for TS cell culture medium" used in the Examples described below.

### <Culture>

TS cells can be cultured by known methods.

Culture conditions can be those generally used for culturing animal cells. A culture temperature can be 30°C to 40°C, is preferably 35°C to 38°C, and is typically 37°C.

A CO₂ concentration can be 2% to 5%, and is typically 5%.

A culture vessel is not particularly limited, and any culture vessel generally used for culturing animal cells can be used. Examples of the culture vessel include culture flasks, culture dishes, and well plates. The culture vessel can be appropriately selected depending on a volume of the culture medium.

Culture can be initiated by seeding TS cells in a culture medium placed in an appropriate culture vessel. A cell density during seeding of TS cells is, for example, 10³ cells/mL to 10¹⁰ cells/mL, and is preferably 10⁴ cells/mL to 10⁸ cells/mL.

The culture may be either adherent culture or suspension culture, with the adherent culture being preferred. When performing the adherent culture, it is preferable to use a culture vessel having a cell-adhesive surface. A surface (for example, a bottom surface) of the culture vessel may be coated with a cell support substrate such as an extracellular matrix (ECM) in order to improve adhesion to TS cells. Examples of the cell support substrate include collagen, gelatin, poly-L-lysine, poly-D-lysine, laminin, and fibronectin. Commercially available extracellular matrix products such as Matrigel (registered trademark) (Corning Life Science) and iMAtrix (registered trademark) 511 may also be used.

A culture period is preferably 1 day or more, and more preferably 2 days or more. An upper limit of the culture period is not particularly limited, and the culture may be continued until confluence. The culture period may be, for example, 1 day to 10 days, may be 2 days to 10 days, or may be 3 days to 10 days. A preferred example of the culture period is 2 days to 3 days.

During the culture period, culture medium replacement may be performed at an interval of approximately 1 day to 3 days (preferably approximately 2 days to 3 days). The culture medium used for the culture medium replacement may be the same as or different from the culture medium before replacement, but is preferably the same. The culture medium before the culture medium replacement obtained by the culture medium replacement may be used as a culture supernatant or may be discarded.

### <Culture Supernatant>

A culture supernatant of TS cells is a liquid part of a culture obtained by culturing TS cells. The culture supernatant of TS cells does not necessarily contain no TS cells, and may contain TS cells. A cell concentration of TS cells contained in the culture supernatant of TS cells is, for example, 0 cells/mL to 10³ cells/mL, is preferably 0 cells/mL to 10² cells/mL, more preferably 0 cells/mL to 10 cells/m, and still more preferably 0 cells/mL to 5 cells/mL. From the viewpoint of maintaining quality and safety, it is preferable that the culture supernatant of TS cells does not contain TS cells (0 cells/mL).

The culture supernatant of TS cells can be obtained from a culture obtained by culturing TS cells. The culture of TS cells may be, for example, a culture of TS cells cultured in a serum-free medium, or a culture of TS cells cultured for 1 day to 10 days. Preferred examples of the culture of TS cells include a culture of TS cells cultured in a serum-free medium for 2 days to 3 days. Preferred examples of the culture supernatant of TS cells include a culture supernatant obtained from a culture of TS cells cultured in a serum-free medium for 2 days to 3 days.

When TS cells are cultured in an adherent manner, the culture supernatant can be obtained by collecting a liquid part (supernatant part) from the culture after removing adherent cells. The collected culture supernatant may be directly used, or may be used after removing TS cells by centrifugation and/or filtration or the like. When centrifugation is performed, the centrifugation may be performed, for example, at a centrifugal acceleration of approximately 300 g to 1000 g for approximately 3 minutes to 15 minutes. The centrifugation is preferably performed at 4°C. When filtration is performed, a 0.22 µm filter can be used. The culture supernatant obtained from the culture of TS cells is preferably centrifuged and then filtered.

When TS cells are cultured in suspension, the culture supernatant can be obtained by centrifuging the culture. After the centrifugation, filtration is preferably performed. Conditions for the centrifugation and the filtration may be the same as those described above.

The culture supernatant of TS cells is preferably stored frozen until use. A storage temperature may be from -80°C to -20°C, with -80°C being preferred.

### <Usage>

The culture supernatant of TS cells is used as an agent for improving an implantation rate. The agent for improving an implantation rate is a drug that has an effect of improving an implantation rate of embryos transferred into the uterus in an assisted reproductive technology. The agent for improving an implantation rate is for use in treating an endometrium before embryo transfer. Treatment on the endometrium with the agent for improving an implantation rate can be achieved by injecting the agent for improving an implantation rate into the uterine cavity.

If the culture supernatant of TS cells has been stored frozen, it is thawed before use. The culture supernatant of TS cells may be used directly, or may be diluted with a pharmaceutically inactive diluent (for example, sterilized purified water, physiological saline, PBS, or the culture medium used for culturing the TS cells) before use.

The subject for which the agent for improving an implantation rate is used is not particularly limited, as long as it is a placentarian in which a placenta is formed during pregnancy. Examples of the placentarian include primates (such as humans, chimpanzees, rhesus monkeys, and marmosets), rodents (such as mice, rats, hamsters, and guinea pigs), and carnivores (such as dogs, cats, and weasels). The subject is preferably a primate, and more preferably a human.

The subject for which the agent for improving an implantation rate is used may be the same as or different from a donor of the cells from which the TS cells used to prepare the culture supernatant of TS cells are derived.

The agent for improving an implantation rate is injected into the uterine cavity of the subject before transferring the embryos into the uterus of the subject in order to improve the implantation rate during the embryo transfer. In this way, the agent for improving an implantation rate can come into contact with the endometrium of the subject, and the endometrium of the subject can be treated with the agent for improving an implantation rate. The agent for improving an implantation rate is preferably injected into the uterine cavity of the subject 1 day to 5 days, more preferably 2 days to 4 days, before transferring the embryos into the uterine cavity of the subject. The injection may be a single time (for example, one time 3 days before the embryo transfer) or a plurality of times of injection may be given every other day or every day. A volume of the agent for improving an implantation rate injected into the uterine cavity may be any amount sufficient to treat the endometrium. The volume of the agent for improving an implantation rate is, for example, 0.01 mL to 1 mL, and preferably 0.05 mL to 0.5 mL.

The embryo used for the embryo transfer may be any of a fertilized egg, a 2-cell stage embryo, a 4-cell stage embryo, an 8-cell stage embryo, a morula, and a blastocyst. The embryo is preferably a blastocyst.

According to the agent for improving an implantation rate of the present embodiment, by injecting the agent into the uterine cavity of the subject before the embryo transfer, the endometrial epithelium can be treated with the agent for improving an implantation rate. In this way, an embryo-receptive capacity of the endometrial epithelium is improved, thereby improving the embryo implantation rate during the embryo transfer. In this way, chances of successful pregnancy in the subject who has received embryo transfer can be improved.

### [Pharmaceutical Composition]

A second aspect of the present disclosure is a pharmaceutical composition for treating or preventing implantation failure. The pharmaceutical composition contains the agent for improving an implantation rate according to the first aspect as an active component.

The pharmaceutical composition of the present embodiment may contain other components in addition to the agent for improving an implantation rate (culture supernatant of TS cells) according to the first aspect. Other components include, for example, a pharmaceutically acceptable carrier. The "pharmaceutically acceptable carrier" refers to a carrier that does not inhibit a physiological activity of the active component and does not show substantial toxicity to the administration subject. The expression "not show substantial toxicity" refers to that a component of the carrier does not show toxicity to the administration subject at a dose that is normally used. In the pharmaceutical composition of the present embodiment, the pharmaceutically acceptable carrier is a carrier that does not inhibit the implantation rate improving activity of the agent for improving an implantation rate according to the first aspect and does not show the substantial toxicity to the administration subject. The pharmaceutically acceptable carrier includes any well-known pharmaceutically acceptable component that is typically considered to be an inactive component. The pharmaceutically acceptable carrier is not particularly limited, but includes, for example, diluents, buffers, vehicles, or the like. The pharmaceutically acceptable carrier may be used alone or in combination of two or more kinds thereof.

The pharmaceutical composition may contain other components in addition to the above component. The other components are not particularly limited, and can use those commonly used in the pharmaceutical field without any particular limitation. Examples of the other components include a pharmaceutical additive other than those described above.

A dosage form of the pharmaceutical composition is not particularly limited, and may be a dosage form generally used as a pharmaceutical formulation. The pharmaceutical composition of the present embodiment is a parenteral preparation that is injected into the uterine cavity. The dosage form of the pharmaceutical composition is preferably a liquid.

The pharmaceutical composition can be administered in a therapeutically effective amount of the agent for improving an implantation rate according to the first aspect. The "therapeutically effective amount" means an amount of a drug effective for treating or preventing a target disease. For example, the therapeutically effective amount of the agent for improving an implantation rate may be an amount effective for treating or preventing implantation failure. The therapeutically effective amount can be, for example, 0.01 mL/individual to 1 mL/individual, or 0.05 mL/individual to 0.5 mL/individual, as an administered amount of the culture supernatant of TS cells.

The pharmaceutical composition may be administered in a single dose or repeatedly. The pharmaceutical composition may be administered only once, for example, 1 day to 5 days, more preferably 2 days to 4 days, before the embryo transfer. Alternatively, the pharmaceutical composition may be administered every other day or every day starting 2 days to 5 days before the embryo transfer.

The pharmaceutical composition of the present embodiment is used to treat or prevent implantation failure. Implantation failure is a condition in which pregnancy does not occur even after a plurality of times of embryo transfer. The pharmaceutical composition of the present embodiment contains the agent for improving an implantation rate according to the first aspect as an active component, and therefore can improve the implantation rate by being injected into the uterine cavity before the embryo transfer. Therefore, implantation failure can be treated or prevented.

### [Method for Producing Agent for Improving Implantation Rate]

A third aspect of the present disclosure is a method for producing an agent for improving an implantation rate. The production method includes (i) a step of culturing trophoblast stem cells, and (ii) a step of obtaining a culture supernatant from a culture obtained by the culturing. The agent for improving an implantation rate according to the first aspect can be produced by the production method according to the present embodiment.

### <Step (i)>

Step (i) can be performed as described above in the section of [Agent for Improving Implantation Rate].

Step (i) is preferably performed in a serum-free medium. The culture period in Step (i) may be 1 day to 10 days.

### <Step (ii)>

Step (ii) can be performed as described above in the section of [Agent for Improving Implantation Rate]. For example, the culture supernatant can be obtained from the culture of TS cells by centrifugation, filtration, and the like.

### <Optional Step>

The production method of the present embodiment may include an optional step in addition to the steps described above. Examples of the optional step include cryopreservation. A temperature for the cryopreservation is, for example, -80°C to -20°C, with -80°C being preferred.

### [Other Embodiments]

In one embodiment, the present disclosure provides a method for improving an implantation rate, including a step of injecting a culture supernatant of TS cells into the uterine cavity of a subject receiving embryo transfer before the embryo transfer.

In one embodiment, the present disclosure provides a method for embryo transfer, including a step of injecting a culture supernatant of TS cells into the uterine cavity of a subject, and a step of transferring embryos into the uterine cavity of the subject.

In the above method, the culture supernatant of TS cells is preferably injected into the uterine cavity 1 day to 5 days before the embryo transfer, more preferably 2 days to 4 days before the embryo transfer.

In one embodiment, the present disclosure provides use of a culture supernatant of TS cells in production of an agent for improving an implantation rate.

In one embodiment, the present disclosure provides use of a culture supernatant of TS cells in production of a pharmaceutical composition for treating or preventing implantation failure.

In one embodiment, the present disclosure provides a culture supernatant of TS cells for use in improving an implantation rate of transferred embryos.

In one embodiment, the present disclosure provides a culture supernatant of TS cells for use in treating or preventing implantation failure.

### Example

The invention will be described below with reference to Example, and the invention is not limited to the following Example.

### <Method>

### 1. Culturing Human TS Cells

Maintenance culture of human TS cells and differentiation induction into ST cells were performed as previously reported (Okae et al. Derivation of Human Trophoblast Stem Cells. Cell Stem Cell (2018), 22(1), 50-63.e6.). A cell line that constitutively expresses an enhanced green fluorescent protein (EGFP) was used to track cells when co-cultured with endometrial epithelial organoids.

### 2. Obtaining Culture Supernatant of Human TS Cells (TS-CM)

Into wells of a 6-well flat-bottom well plate (IWAKI Microplate, AGC Technoglass Co., Ltd.), 1 µL/well of i-Matrix (registered trademark) 511 (Nippi Corporation) was placed and allowed to stand for 10 minutes. In this way, inner walls of the wells were coated with i-Matrix. In the coated well plate, 2 mL/well of a basal medium for a TS cell culture medium or the DMEM/F-12 medium was placed. Human TS cells were seeded at a cell density of 1 × 10⁵ cells/well and cultured as adherent cells at 37°C and a CO₂ concentration of 5% for 2 days or 3 days, followed by washing with PBS. Thereafter, replacement with the basal medium for the TS cell culture medium or the DMEM/F-12 medium was performed, and the cells were cultured at 37°C and a CO₂ concentration of 5% for 2 days. Thereafter, a supernatant was collected and centrifuged at 500 g for 5 minutes, and the centrifuged supernatant was passed through a 0.22 µm filter and stored at -80°C until use. To obtain ST-CM, the TS cells were induced to differentiate into ST cells using a previously reported differentiation induction method (Okae et al. Derivation of Human Trophoblast Stem Cells. Cell Stem Cell (2018), 22(1), 50-63.e6.), and the supernatant was collected in the same manner as above.

A composition of the basal medium for the TS cell culture medium (TS basal medium) is as follows. DMEM/F-12 (Life Technologies) supplemented with 1% ITS-X supplement (FUJIFILM Wako), 0.15% bovine serum albumin (BSA) (FUJIFILM Wako), 1% Knockout Serum Replacement (KSR, Thermo Fisher Scientific), 200 mM L-ascorbic acid (FUJIFILM Wako), 5,000 units/mL penicillin, and 5,000 µg/mL streptomycin

### 2. Culturing Human Endometrial Epithelial Organoids

Endometrial epithelial organoids were established as previously reported (Turco et al. Long-term, hormone-responsive organoid cultures of human endometrium in a chemically defined medium. Nature Cell Biology (2017) 19(5), 568-577.). A decidual tissue from an aborted woman 6 weeks to 8 weeks pregnant who had given informed consent was cut into small pieces, immersed in an enzyme solution (Collagenase V/Dispase II), and reacted at 37°C for 1 hour while shaking. Thereafter, a cell fraction trapped in a cell strainer (100 µm) was embedded in Matrigel and cultured. Culture medium replacement was performed every 2 days to 3 days. During sub-culturing, the cells were detached from the culture plate using the basal medium for the TS cell culture medium and fragmented by pipetting. The fragmented organoids were diluted, embedded in Matrigel, and subcultured.

### 3. Production of Human Endometrial Model

The fragmented human endometrial epithelial organoids were suspended in a matrix made by mixing collagen gel and Matrigel (registered trademark) (Corning) (collagen gel:Matrigel = 7:3 (volume ratio)). Droplets of the suspension were left to stand in a 48-well plate for 30 minutes to solidify, and then cultured in the ESCY medium for 7 days to 8 days. Then, estradiol (E₂ (10 nM) was added and cultured for another 2 days, and then E₂ (10 nM), medroxyprogesterone acetate (MPA) (1 µM), and 8-bromo-cAMP (50 µM) were added to perform maturation.

A composition of the ESCY medium is as follows.

DMEM/F-12 (Life Technologies) supplemented with 1% ITS-X supplement, 0.15% BSA (FUJIFILM Wako) 1% Knockout Serum Replacement (KSR, Thermo Fisher Scientific), 200 mM L-ascorbic acid (FUJIFILM Wako), 50 ng/ml EGF, 2 µM CHIR99021, 2 µM SB202190, and 10 µM Y27632

### 4. Immunostaining

Cells and organoids were fixed with 4% paraformaldehyde (PFA), washed with PBS, and then nonspecific reactions were blocked for 1 hour with PBS containing 5% normal goat serum and 0.3% Triton X-100. Next, a primary antibody was added, and after reaction at 4°C overnight, the cells were stained with a fluorescently labeled secondary antibody (Cell Signaling) and Hoechst 33342 (Dojindo Chemical). Observation was performed using BZ-X810 (Keyence) and LSM 710 (Carl Zeiss).

### 5. Quantitative Real-time RT-PCR

RNA was extracted and purified from cells and organoids using the RNeasy Mini Kit (QIAGEN). cDNA was prepared using PrimeScript II (Takara Bio), and a real-time PCR reaction was performed using StepOnePlus Real-Time PCR System (Applied Biosystems) and TB Green Premix Ex TaqTM II (Takara Bio).

### 6. RNA-sequencing

RNA was extracted and purified from the cells and organoids using RNeasy Mini Kit (QIAGEN), and then genomic DNA was digested with DNase I (QIAGEN). An RNA library was prepared using the NEBNext (registered trademark) Ultra^{™} II Directional RNA Library Prep Kit (New England Biolabs), and sequence data was obtained by reading 150-bp paired-end reads on the Illumina NovaSeq 6000 platform (Illumina). The sequence data were trimmed using TrimGalore and aligned to the reference genome (UCSC hg38) using STAR. An expression level (TPM) of genes was calculated using RSEM. Differentially expressed genes were extracted using DESeq2 software. GO term analysis was performed using clusterProfiler.

### 7. Induction of Blastoid from Human ES Cells

A blastocyst-like structure (Blastoid) was prepared from naive ES cells as previously reported (Yu et al. Blastocyst-like structures generated from human pluripotent stem cells. Nature (2021) 591, 620-626.).

### 8. In Vitro Implantation Assay

Ishikawa cells (endometrial epithelial cells) were seeded in a 96-well plate and cultured in a 10% FBS-containing DMEM medium (Life Technologies) at 37°C until confluence. In a treated group, the culture medium was replaced with TS-CM and the cells were cultured for 48 hours. In an untreated group, the cells were cultured for the same period without replacing the culture medium. Thereafter, the blastocyst-like structure (Blastoid) prepared from naive pluripotent stem cell-type ES cells was added to the treated group and the untreated group, and co-cultured at 37°C. An adhesion rate was quantified 5 hours after the co-culturing of the blastocyst-like structures (Blastoid) prepared from the naive ES cells in the treated group and the untreated (control) group.

### <Experiment Results>

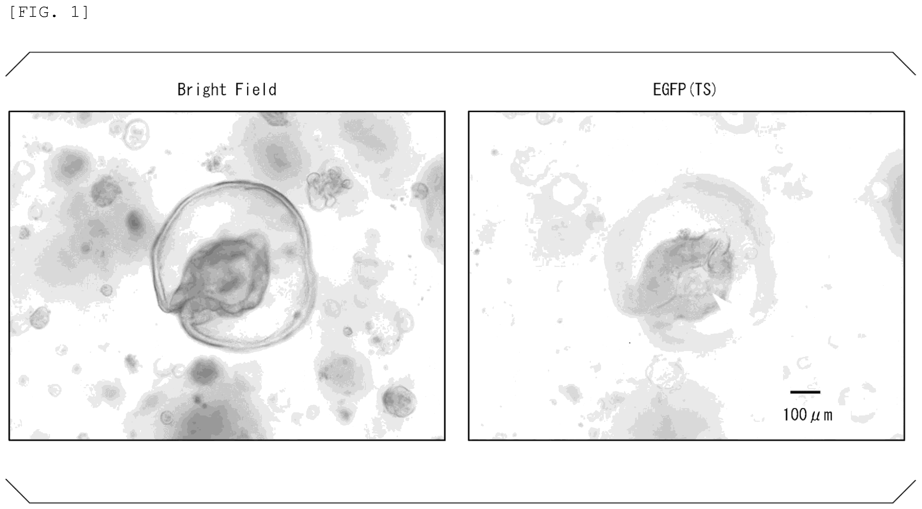

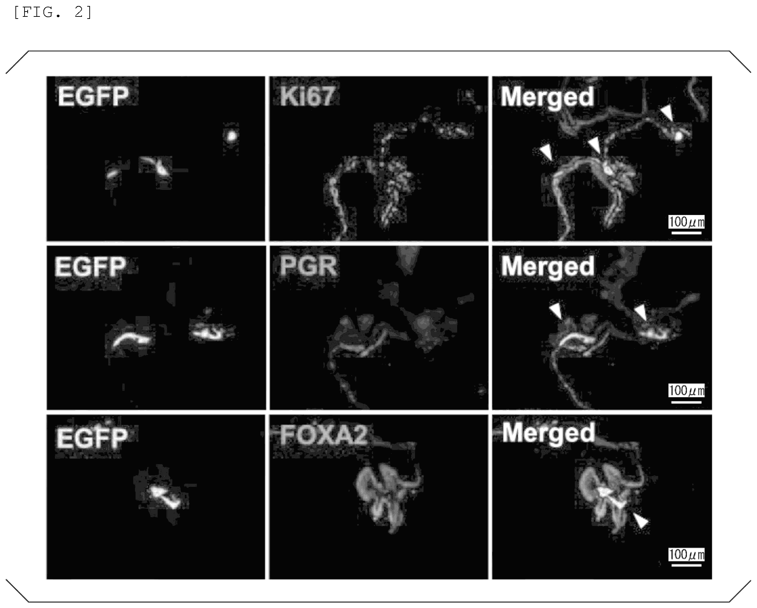

In order to develop an infertility treatment using human TS cells, an effect of TS cells on endometrial epithelial cells was investigated. When TS cells (GFP-TS) that constitutively express EGFP were co-cultured with endometrial epithelial organoids (EMOs) in Matrigel, the epithelial cell layer in contact with the TS cells thickened (FIG. 1). It was confirmed by immunostaining that the endometrial epithelial cells in contact with TS cells were positive for a proliferative cell marker Ki67, a hormone-responsive PGR, and a glandular epithelial marker FOXA2 (FIG. 2). Therefore, it was suggested that the contact with TS cells induces cell proliferation, hormone response, and glandular epithelial differentiation in the endometrial epithelial cells.

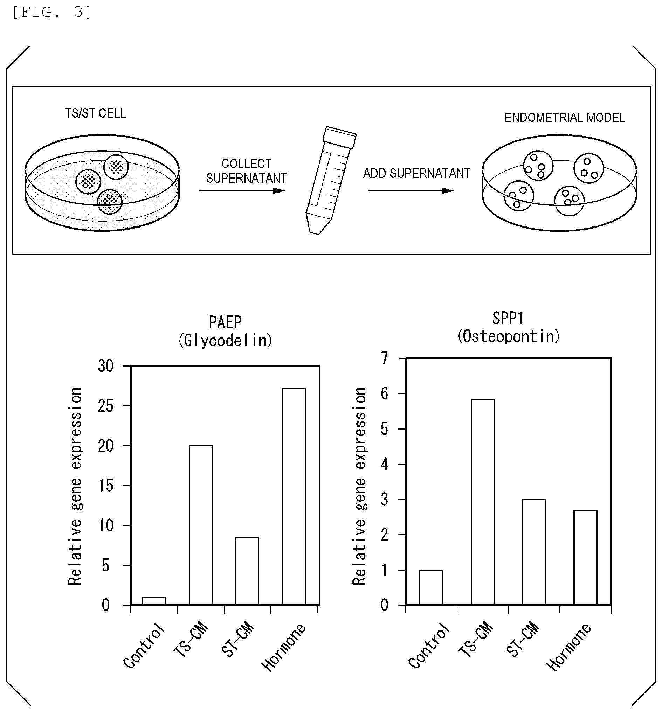

Next, whether this effect was due to direct contact or humoral factors was confirmed. To the endometrial model prepared in the above section "3. Production of Human Endometrial Model", 250 µL of the culture supernatant of TS cells (TS-CM) was added, and gene expression analysis was performed. In the endometrial model treated with TS-CM, the expression of both implantation stage markers PAEP and SPP1 increased (FIG. 3).

In FIG. 3, "ST-CM" indicates that 250 µL of a culture supernatant of ST cells (ST-CM) was added to the endometrial model instead of TS-CM. "Hormone" indicates that instead of TS-CM, EPCP (E₂ (10 nM), MPA (1 µM), 8-Bromo-cAMP (50 µM), and prolactin (50 mg/mL)) was added. Control indicates that none of the above treatments has been done (untreated).

In the endometrial model treated with ST-CM, the expression of PAEP and SPP1 was increased as compared with Control, but a rate of increase in expression was lower than that in the group treated with TS-CM.

In the endometrial model treated with Hormone, the expression of PAEP and SPP1 was increased as compared with Control, but a rate of increase in expression of SPP1 was lower than that in the groups treated with TS-CM and ST-CM.

From the above results, it was inferred that TS-CM is highly effective in transitioning the endometrium to the implantation stage.

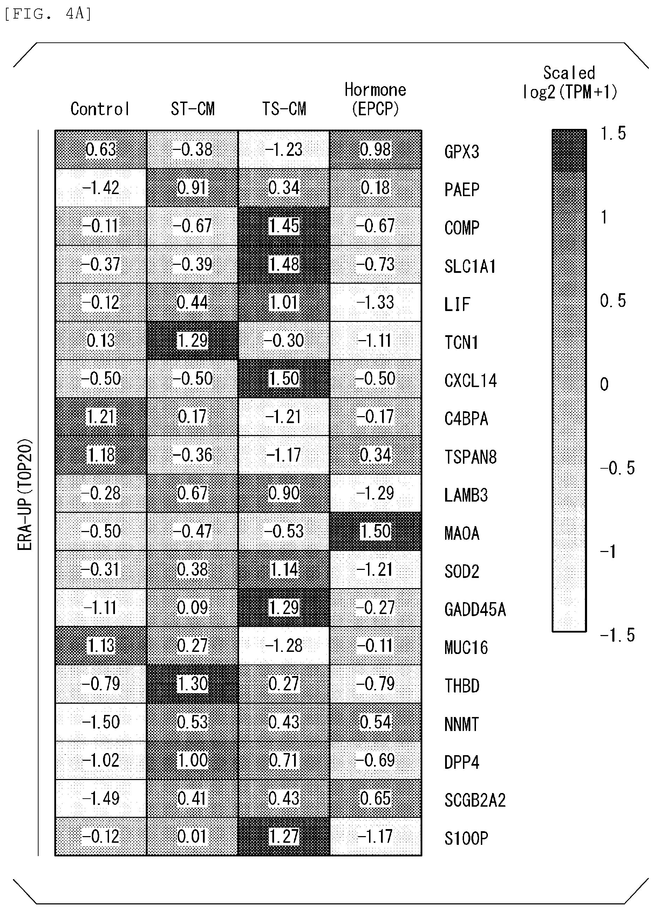

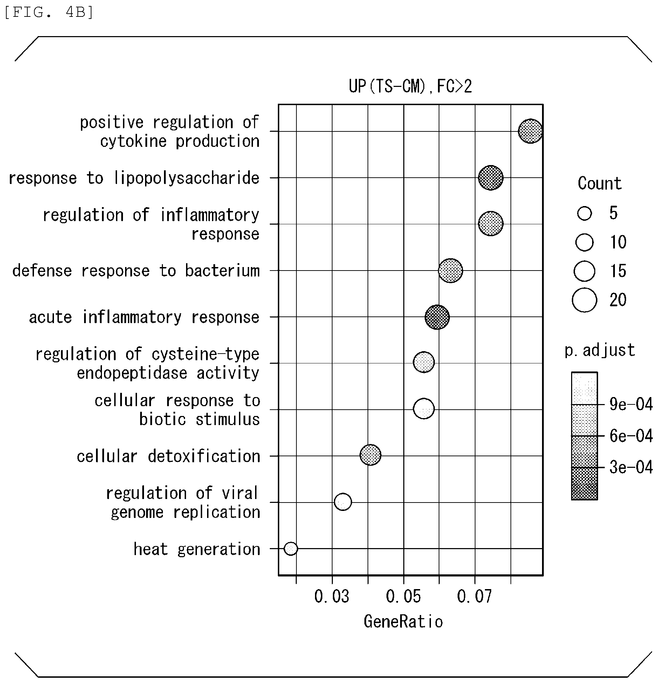

RNA was extracted from the endometrial model treated with TS-CM, ST-CM, or Hormone (EPCP) and subjected to RNA-seq analysis. In the RNA-seq analysis, the expression of top 20 genes (ERA-UP (TO20)) that increase during the implantation period used in the endometrial receptivity analysis (ERA) was examined. TS-CM treatment increased the expression of most of the genes (13 genes out of 20 genes). GO term analysis revealed that terms related to signals that increase in the endometrial epithelium during the early stages of implantation, such as "regulation of inflammatory response", were enriched (FIGS. 4A and 4B).

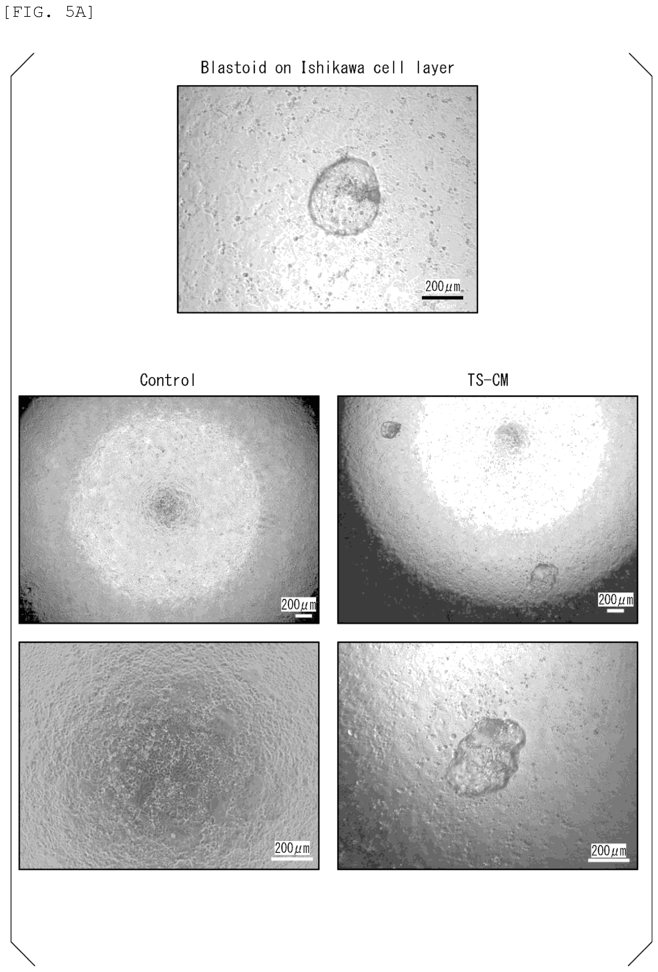

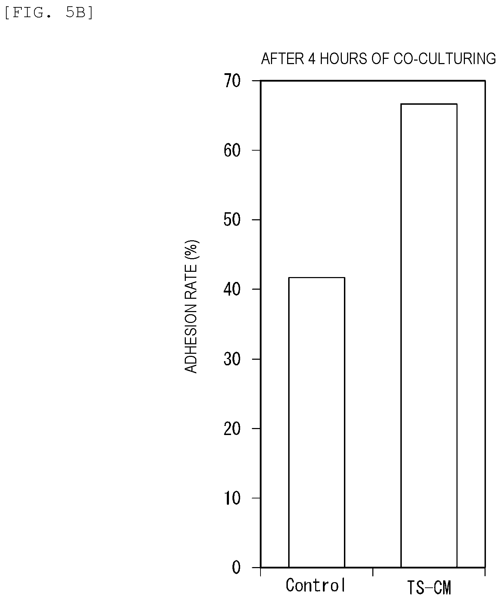

Next, an implantation-promoting effect of TS-CM was examined using an implantation assay (see "8. In Vitro Implantation Assay" above) using Blastoid adhesion to the apical surface of the plate-cultured endometrial epithelial cells (Ishikawa) as an indicator. By pretreating the endometrial epithelial cells (Ishikawa) with TS-CM, the adhesion rate of Blastoid after 4 hours of co-culturing increased 1.6 times as compared with the untreated group (Control) (FIG. 5).

These results confirmed that TS-CM has the effect of improving the embryo-receptive capacity of the endometrial epithelium. Therefore, it was suggested that factors secreted by TS cells improve the embryo-receptive capacity of the endometrial epithelium.

### Industrial Applicability

According to the present invention, there are provided an agent for improving an implantation rate that can be easily produced, a pharmaceutical composition containing the agent for improving an implantation rate, and a method for producing the agent for improving an implantation rate.

The preferred Examples of the invention has been described above, but the invention is not limited to these Examples. In a scope not departing from the gist of the invention, additions, omissions, replacements, and other changes of the configuration can be made. The invention is not limited to the above description, and is limited only by the appended claims.

## Claims

1. An agent for improving an implantation rate, the agent comprising:
a culture supernatant of a trophoblast stem cell.

2. The agent for improving an implantation rate according to claim 1, which is for use in treating an endometrium before embryo transfer.

3. The agent for improving an implantation rate according to claim 1 or 2, wherein
the culture supernatant is a culture supernatant obtained from a culture of the trophoblast stem cell cultured in a serum-free medium.

4. The agent for improving an implantation rate according to claim 1 or 2, wherein
the culture supernatant is a culture supernatant obtained from a culture of the trophoblast stem cell cultured for 1 day to 10 days.

5. A pharmaceutical composition for treating or preventing implantation failure, the pharmaceutical composition comprising:
the agent for improving an implantation rate according to claim 1 or 2.

6. A method for producing an agent for improving an implantation rate, the method comprising:
(i) a step of culturing a trophoblast stem cell; and
(ii) a step of obtaining a culture supernatant from a culture obtained by the culturing.

7. The method for producing an agent for improving an implantation rate according to claim 6, wherein
the culturing in the step (i) is performed in a serum-free medium.

8. The method for producing an agent for improving an implantation rate according to claim 6 or 7, wherein
the culturing in the step (i) is performed for 1 day to 10 days.
